# EUROPEAN PATENT SPECIFICATION

(11) **EP 0 496 629 B1**
(45) Date of publication and mention of the grant of the patent: **28.12.1994**
(21) Application number: 92300605.0
(22) Date of filing: 24.01.1992
(51) Int. Cl.: F04B 43/04, A61M 5/142

(54) **Pump apparatus for biomedical use**
Pumpe zur biomedizinischen Verwendung
Pompe pour utilisation biomédicale

(30) Priority: 25.01.1991 GB 9101648
(43) Date of publication of application: 29.07.1992
(73) Proprietor: BESPAK PLC, King's Lynn Norfolk PE30 2JJ (GB)
(72) Inventor: Ross, Calvin John, Suffolk, IP27 ORB (GB); Humberstone, Victor Carey, Cambridge, CB2 5BT (GB); Warby, Richard John, Cambridgeshire, P13 2BP (GB)
(74) Representative: Flegg, Christopher Frederick

(56) References cited:
- EP-A- 0 398 583
- WO-A-87/03342
- US-A- 3 425 415
- US-A- 4 456 009
- US-A- 4 798 589

## Description

This invention relates to pump apparatus for biomedical use and in particular but not exclusively for use in intravenous infusion of liquids to the human or animal body.

It is known from EP0398583, which is considered to be the closest prior art document,to provide a pump apparatus having a pump body defining a pump chamber which is variable in volume by movement of a displaceable wall of the pump body. The pump body is releasably connected to a housing comprising a piezoelectric transducer which is operable when the housing and pump body are connected to reciprocatingly move the displaceable wall so as to provide pumping action.

In order to ensure effective pumping action it is necessary for the displaceable wall to be biassed into continuous contact with the piezoelectric transducer which is operable to flex both towards and away from the pump chamber. The pump body is also required to be a disposable single use item whereas the housing contains electronic and other components which are re-usable with further pump bodies. Successive pump bodies must therefore be releasably connectable to the housing in a manner which provides accurate alignment with the piezoelectric transducer and which achieves the optimum bias of the displaceable wall in contact with the transducer.

According to the present invention there is disclosed pump apparatus comprising a pump body defining a pump chamber, a displaceable wall of the pump body being displaceable to vary the volume of the pump chamber, biassing means biassing the displaceable wall into a fully extended position in which the volume of the chamber is maximised, a housing, connecting means releasably connecting the housing to the pump body, the housing comprising a piezoelectric transducer operable to reciprocatingly move the displaceable wall when the housing and the pump body are connected, the connecting means being operable between the pump body and the housing during connection of the pump body to the housing to move the pump body from a first position relative to the housing in which the displaceable wall is in its fully extended position in abutment with the transducer and a second position relative to the housing in which the movable wall is partially extended in its normal operating position whereby the displaceable wall is biassed into contact with the transducer by action of the biassing means such that contact is maintained during reciprocation of the transducer, characterised by the connecting means comprising cam means and at least one locking member connected to the housing and movable between an unlocked position and a locking position in which a foot portion of the locking member overlays a contact surface of the pump body so as to prevent disconnection of the pump body from the housing, and wherein at least one of the foot portion and the contact surface comprises a ramped surface such that movement of the locking member into the locking position provides cam action whereby the foot portion and the contact surface together constitute the cam means.

An advantage of such pump apparatus is that during connection of the pump body to the housing the displaceable wall is depressed from its fully extended position to its partially extended position in a controlled manner through a displacement which can be accurately controlled to a predetermined value. It is particularly important where the biassing force exerted on the displaceable wall is proportional to the displacement of the displaceable wall from its fully extended position so that any error in the displacement relative to the preferred predetermined value will result in the biassing force being either too great or too little for the provision of effective pumping action.

Preferable the pump apparatus comprises a plurality of locking members which are peripherally spaced relative to the displaceable wall, the locking members being mounted on a mounting member so as to be movable in unison between their respective locking and unlocked positions.

An advantage of this arrangement is that the peripheral spacing of the locking members ensures that an evenly distributed load is applied to the pump body both during the locking movement and during the continued connection of the housing to the pump body to thereby avoid possible distortion and imperfect connection to the housing.

Conveniently the mounting member comprises an annular flange and the housing further comprising annular guide means cooperating with the flange to facilitate axial rotation of the annular flange between positions corresponding to the unlocked and locking positions of the locking members.

Preferably the pump body is provided with at least one aperture through which a co-operating locking member extends when the housing is connected to the pump body.

Such an arrangement assists in indicating to the user the correct positioning of the body relative to the housing during connection and assists in achieving alignment between the displaceable wall and the transducer.

Preferably the displaceable wall is formed of an elastomeric material and includes a relatively thick central portion which is relatively rigid and a relatively flexible annular membrane portion connected peripherally to the rigid portion, the membrane portion having a shape memory such that the central portion is biassed into the fully extended position to thereby constitute the biassing means.

A preferred embodiment of the present invention will now be described by way of example only and with reference to the accompanying drawings of which:-
Figure 1 is a perspective view of a pump apparatus having a pump body operatively connected to a housing;
Figure 2 is a plan view of the pump apparatus of Figure 1;
Figure 3 is a section taken at II-II of the pump apparatus of Figures 1 and 2;

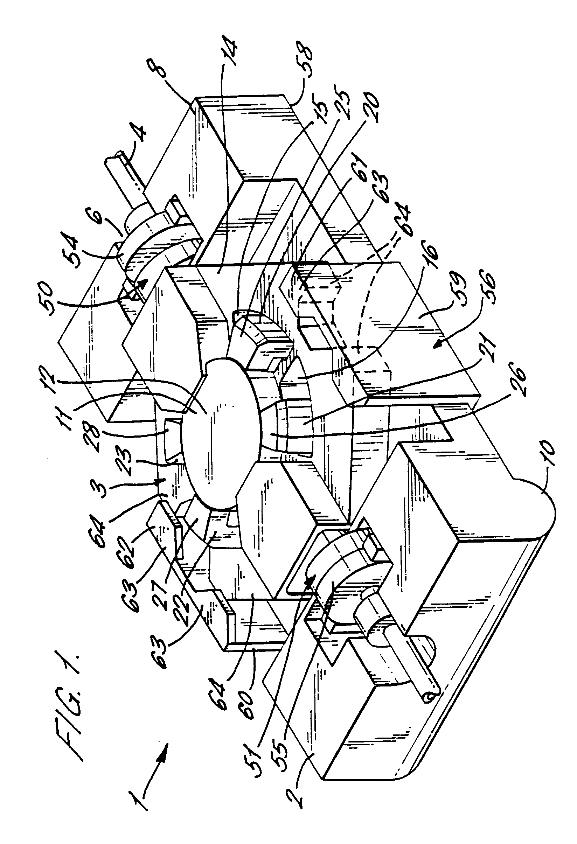

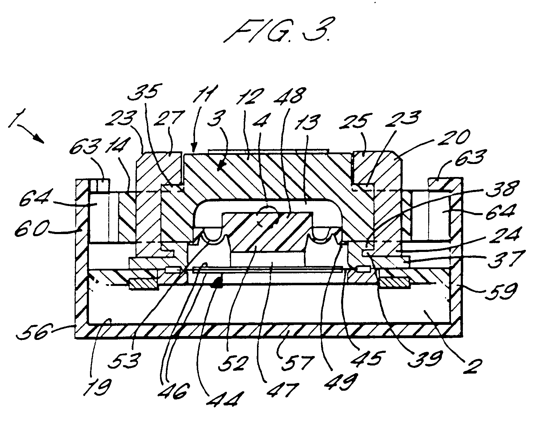

Figure 3 is a partially sectioned side elevation of the pump apparatus of Figures 1 to 3; and

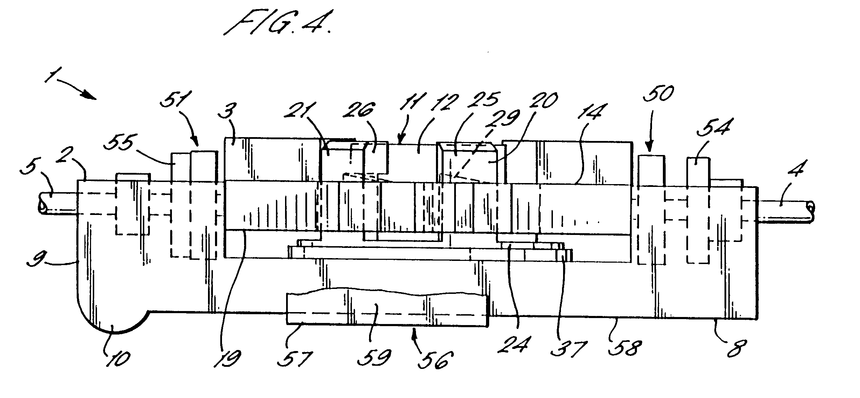

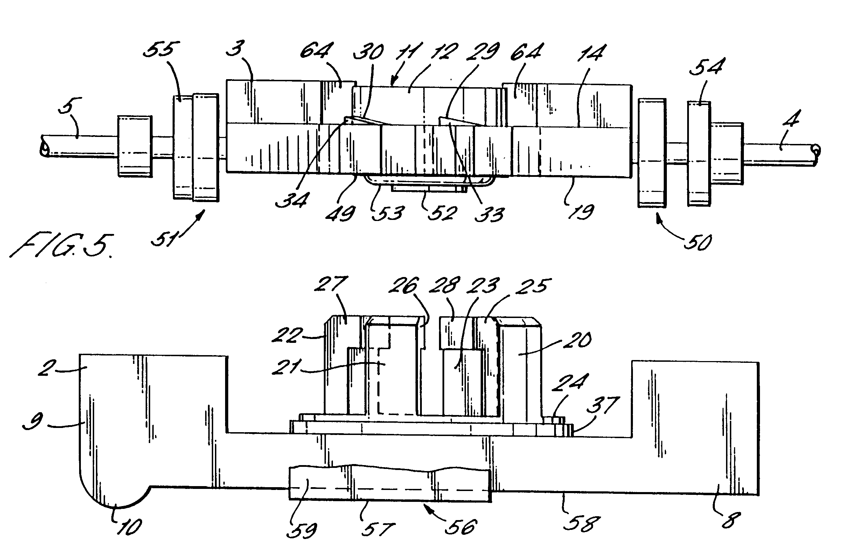

Figure 5 is a side view of the pump apparatus of Figures 1 to 4 with the pump body disconnected from the housing.

In Figure 1 a pump apparatus 1 has a housing 2 which is operatively connected to a pump body 3.

The pump body 3 has an inlet duct 4 and an outlet duct 5 which are received in first and second recesses 6 and 7 provided in first and second end portions 8 and 9 of the housing 2 respectively. The second end portion 9 includes a battery compartment 10 for powering electronic circuitry (not shown) contained within the housing 2.

The pump body 3 includes a centrally located cylindrical portion 11 projecting at right angles to the inlet and outlet ducts 4 and 5 and constituting a rigid wall 12 of a chamber 13 as shown in Figure 3.

The cylindrical portion 11 projects from a planar upper surface 14 of the pump body 3 and first, second, third and fourth part annular apertures 15, 16, 17 and 18 are circumferentially spaced peripherally of the cylindrical portion 11 so as to communicate between the upper surface 14 and a lower surface 19 of the pump body.

First, second, third and fourth locking members 20, 21, 22 and 23 project upwardly from an annular mounting member 24 to which they are rigidly connected and project through the respective apertures 15 to 18. The locking members 20 to 23 include respective first, second, third and fourth foot members 25, 26, 27 and 28 which project radially inwardly with respect to the cylindrical portion 11 and, in the connected configuration shown in Figures 1 to 4, overlay respective cam surfaces 29 to 32 defined by cam formations 33 to 36 of the pump body 3.

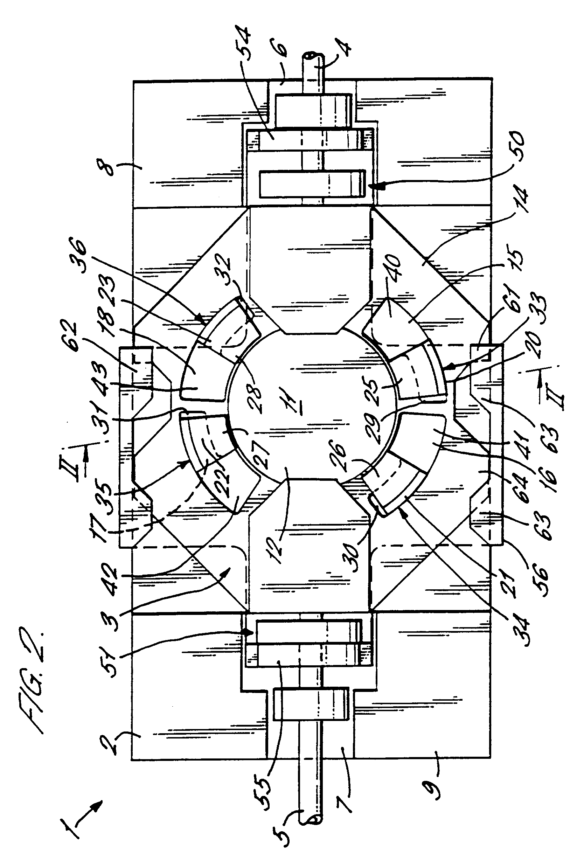

The cam surfaces 29 to 32 are ramped relative to the upper surface 14 such that rotation of the mounting member 24 in a clockwise direction as viewed in Figure 2 results in the foot members 25 to 28 contacting progressively thicker portions of cam formations 33 to 36.

The mounting member 24 is retained in sliding contact with the housing 2 by means of an annular guide 37 which has a peripheral groove 38. The mounting member 24 has an annular flange 39 which projects radially inwardly into the groove 38 such that the mounting member is slidable in a circumferential direction relative to the guide 37.

The apertures 15 to 18 include respective enlarged portions 40 to 43 which are angularly offset from the cam formations 33 to 36 and are of sufficient size to allow the foot members 25 to 28 respectively to pass through the respective apertures 15 to 18.

As shown in Figure 3 the housing 2 includes a circular piezoelectric transducer 44 of 20 mm diameter which comprises a brass disc 45 sandwiched between ceramic wafers 46. A centrally located boss 47 of a plastics material is secured to the piezoelectric transducer 44.

The pump body 3 includes a deformable wall 48 which is formed of an elastomeric material. The deformable wall 8 has an annular rim 49 which is bonded to the lower surface 19 of the pump body 3 so as to form a closure to the chamber 13 which is defined between the deformable wall 48 and the rigid wall 12. The inlet and outlet ducts 4 and 5 communicate with the chamber 13 for the inlet and outlet of fluid and inlet and outlet non-return valves 50 and 51 respectively are disposed in the inlet and outlet ducts so as to permit fluid flow in a direction from the inlet duct to the outlet duct.

The deformable wall 48 includes a relatively thick central portion 52 which is rigid and a relatively flexible annular membrane portion 53 connected peripherally to the rigid portion, the membrane portion being much thinner than the central portion so as to be resiliently deformable.

The membrane portion 53 is moulded so as to have a shape memory such that the central portion is biassed into a fully extended position as shown in Figure 5. Displacement of the central portion 52 in a direction towards the rigid wall 12 is accommodated by flexure of the membrane portion 53. As shown in Figure 3 the central portion 52 is depressed to a partially extended position when the pump body 3 is operatively connected to the housing 2 and in this position the central portion 52 abuts the boss 47 of the piezoelectric transducer 44. The predetermined value for the displacement of the central portion during this depression is 0.3 mm.

The inlet and outlet ducts 4 and 5 are provided with annular magnets 54 and 55 through which they respectively extend and the inlet and outlet valves 50 and 51 comprise ferrous valve members (not shown) such that the operation of the valves is dependent on the axial position of the magnets 44 and 45. The housing 2 is provided with a longitudinally slidable handle 56 which, when the housing 2 is connected to the pump body 3, engages the magnets 54 and 55 such that the magnets can be moved between the normal operating position in which the valve members are biassed into contact with respective valve seats (not shown) and a priming position in which both of the valves are held open to permit the free flow of fluid through the pump body.

The handle 56 comprises a base plate 57 which is maintained in sliding contact with a lower surface 58 of the housing 2 and further includes side plates 59 and 60 which project at right angles to the base plate to the level of the upper surface 14 of the pump body. The side plates 59 and 60 include inturned edge portions 61 and 62 respectively each having projecting tabs 63 which overlay laterally projecting lugs 64 of the pump body 3 to thereby hold in place the pump body 3 relative to the housing 2 when operatively connected as shown in Figure 1. When the handle is moved to its priming position (not shown) the tabs 63 interdigitate with the lugs 64 such that the handle no longer holds the pump body in place relative to the housing 2.

In use to commence an infusion of a liquid to a patient, a fresh pump body (supplied in a sterile package) is connected to suitable tubing such that a reservoir of fluid is connected to the inlet duct 4 and a cannulation device is connected to the outlet duct 5. The pump body 3 is oriented such that the outlet duct extends vertically downwards and in this position the magnets 54 and 55 fall under gravity to a priming position in which the inlet and outlet valves 50 and 51 are held opened. The pump body is then primed by allowing fluid to flow through the chamber 13.

After priming the tubing is clamped and the pump body 3 is then presented to the housing 2 as shown in Figure 5, the pump body 3 and the housing 2 then being brought together so that the locking members 20 to 23 pass through the apertures 15 to 18 respectively. The housing 2 and the pump body 3 are moved together until contact is made between the boss 47 of the piezoelectric transducer 44 and the central portion 52 of the deformable wall 48. The locking members 20 to 23 are then manually rotated relative to the housing 2 in a clockwise direction as viewed in Figure 1 so that the foot members 25 to 28 are translated circumferentially relative to the cylindrical portion 11 and engage the cam surfaces 29 to 32 respectively.

Continued rotation of the locking members 20 to 23 results in cam action between the cam formations 33 to 36 and the foot members 25 to 28 whereby the pump body 3 is progressively advanced further towards the housing 2. During this further advancement the membrane portion 53 of the deformable wall 48 flexes to accommodate depression of the central portion 52 from its fully extended position to its partially extending position as shown in Figure 3. The piezoelectric transducer is considerably stiffer than the deformable wall 48 to an extent such that no discernable deflection of the transducer takes place during this further advancement. Because of the shape memory of the membrane portion 53, this depression of the central portion 52 results in the central portion being biassed into positive contact with the piezoelectric transducer 44.

The handle 56 is then longitudinally advanced from its priming position to the normal operating position shown in Figure 1. In this position the tabs 63 overlay the lugs 64 to positively secure the pump body 3 relative to the housing 2.

The sliding motion is also sensed by means of a sensor (not shown) to activate the electronic circuitry of the housing 2. Pumping action is then commenced by actuation of the piezoelectric transducer 44 to cyclically flex the transducer towards and away from the chamber 13 with a displacement of ± 10 microns. The movement of the transducer 44 is followed precisely by the deformable wall 48 which is biassed into continuous contact with the transducer so that the volume of the chamber 13 is cyclically varied to produce pump action.

On completion of an infusion the pump body 3 is separable from the housing 2 by moving the handle 56 into its priming position and counter-rotating the locking members 20 to 23 so that the pump body 3 can be lifted from the housing 2. The pump body 3 will typically be discarded as a disposable item and the housing 2 will be reused with further sterile pump bodies in further infusion operations.

The preferred material of the displaceable wall is Hytrel (registered trade mark) although other elastomers may be used.

Alternative embodiments are envisaged in which the cam means is constituted by cam formations on the locking members in addition to or in place of the cam formations of the pump body.

The valves 50 and 51 may alternatively be non-magnetic non-return valves which are biassed into a closed position and are provided with an alternative means of priming which may similarly comprise a means of holding the valves open during the priming operation.

## Claims

1. Pump apparatus (1) comprising a pump body (3) defining a pump chamber (13), a displaceable wall (48) of the pump body being displaceable to vary the volume of the pump chamber, biassing means (53) biassing the displaceable wall into a fully extended position in which the volume of the chamber is maximised, a housing (2), connecting means (20-23,25-28,33-36) releasably connecting the housing to the pump body, the housing comprising a piezoelectric transducer (44) operable to reciprocatingly move the displaceable wall when the housing and the pump body are connected, the connecting means being operable between the pump body and the housing during connection of the pump body to the housing to move the pump body from a first position relative to the housing in which the displaceable wall is in its fully extended position in abutment with the transducer and a second position relative to the housing in which the movable wall is partially extended in its normal operating position whereby the displaceable wall is biassed into contact with the transducer by action of the biassing means such that contact is maintained during reciprocation of the transducer, characterised by the connecting means comprising cam means (25-28, 33-36) and at least one locking member (20-23) connected to the housing and movable between an unlocked position and a locking position in which a foot portion (25-28) of the locking member overlays a contact surface (29-32) of the pump body so as to prevent disconnection of the pump body from the housing, and wherein at least one of the foot portion and the contact surface comprises a ramped surface (29-32) such that movement of the locking member into the locking position provides cam action whereby the foot portion and the contact surface together constitute the cam means.

2. Pump apparatus as claimed in claim 1 comprising a plurality of locking members which are peripherally spaced relative to the displaceable wall, the locking members being mounted on a mounting member (24) so as to be movable in unison between their respective locking and unlocked positions.

3. Pump apparatus as claimed in claim 2 wherein the mounting member comprises an annular flange (39) and the housing further comprising annular guide means (37) cooperating with the flange to facilitate axial rotation of the annular flange between positions corresponding to the unlocked and locking positions of the locking members.

4. Pump apparatus as claimed in any preceding claim wherein the pump body is provided with at least one aperture (15-18) through which a cooperating locking member extends when the housing is connected to the pump body.

5. Pump apparatus as claimed in any preceding claim wherein the displaceable wall is formed of an elastomeric material and includes a relatively thick central portion (52) which is relatively rigid and a relatively flexible annular membrane portion (53) connected peripherally to the rigid portion, the membrane portion having a shape memory such that the central portion is biassed into the fully extended position to thereby constitute the biassing means.

## Patentansprüche

1. Pumpvorrichtung (1), umfassend einen Pumpenkörper (3), welcher eine Pumpenkammer (13) begrenzt, wobei eine verlagerbare Wandung (48) des Pumpenkörpers zur Veränderung des Pumpenkammervolumens verlagerbar ist, Vorspannmittel (53), welche die verlagerbare Wandung in eine vollausgefahrene Stellung vorspannen, in der das Kammervolumen maximiert ist, ein Gehäuse (2), Verbindungsmittel (20-23, 25-28, 33-36), welche das Gehäuse lösbar mit dem Pumpenkörper verbinden, wobei das Gehäuse einen piezoelektrischen Wandler (44) umfaßt, der bei mit dem Pumpenkörper verbundenem Gehäuse zur Hin- und Herbewegung der verlagerbaren Wandung betreibbar ist, wobei die Verbindungsmittel während der Verbindung des Pumpenkörpers mit dem Gehäuse dahingehend zwischen dem Pumpenkörper und dem Gehäuse wirken können, den Pumpenkörper aus einer ersten Relativstellung zum Gehäuse, in der sich die verlagerbare Wandung in ihrer vollausgefahrenen Stellung befindet, in Anlage an den Wandler und einer zweiten Relativstellung zum Gehäuse, in der die bewegliche Wandung teilausgefahren ist, in seine normale Betriebsstellung zu bewegen, wobei die verlagerbare Wandung durch die Wirkung der Vorspannmittel derart in Kontakt mit dem Wandler vorgespannt wird, daß der Kontakt während einer Hin- und Herbewegung des Wandlers aufrechterhalten wird,
**dadurch gekennzeichnet, daß**
die Verbindungsmittel Nockenmittel (25-28, 33-36) sowie wenigstens ein Arretierelement (20-23) umfassen, welches mit dem Gehäuse verbunden ist und zwischen einer nicht arretierten Stellung und einer arretierten Stellung beweglich ist, in welcher arretierten Stellung ein Fußabschnitt (25-28) des Arretierelements zur Vermeidung einer Trennung des Pumpenkörpers vom Gehäuse über einer Kontaktfläche (29-32) des Pumpenkörpers liegt, und wobei der Fußabschnitt oder/und die Kontaktfläche eine Rampenfläche (29-32) umfassen, derart, daß eine Bewegung des Arretierelements in die arretierte Stellung zu einer Nockenwirkung führt, wobei der Fußabschnitt und die Kontaktfläche zusammen die Nockenmittel bilden.

2. Pumpvorrichtung nach Anspruch 1, umfassend eine Mehrzahl Arretierelemente, welche bezüglich der verlagerbaren Wandung umfangsmäßig Abstand voneinander aufweisen, wobei die Arretierelemente an einem Befestigungselement (24) angebracht sind, so daß sie gemeinsam zwischen ihren jeweiligen arretierten Stellungen und nicht arretierten Stellungen beweglich sind.

3. Pumpvorrichtung nach Anspruch 2, bei der das Befestigungselement einen Ringflansch (39) umfaßt und das Gehäuse ferner ringförmige Führungsmittel (37) umfaßt, welche zur Erleichterung einer axialen Drehung des Ringflansches zwischen Stellungen, welche den nicht arretierten Stellungen und den arretierten Stellungen der Arretierelemente entsprechen, mit dem Flansch zusammenwirken.

4. Pumpvorrichtung nach einem der vorhergehenden Ansprüche, bei der der Pumpenkörper mit wenigstens einer Öffnung (15-18) versehen ist, durch welche sich ein zusammenwirkendes Arretierelement erstreckt, wenn das Gehäuse mit dem Pumpenkörper verbunden ist.

5. Pumpvorrichtung nach einem der vorhergehenden Ansprüche, bei der die verlagerbare Wandung aus einem Elastomermaterial gebildet ist und einen vergleichsweise dicken Mittelabschnitt (52), welcher vergleichsweise starr ist, sowie einen vergleichsweise flexiblen, ringförmigen Membranabschnitt (53) umfaßt, welcher mit dem starren Abschnitt am Umfang verbunden ist, wobei der Membranabschnitt ein Formerinnerungsvermögen hat, derart, daß der Mittelabschnitt in die vollausgefahrene Stellung vorgespannt ist, um dadurch die Vorspannmittel zu bilden.

## Revendications

1. Pompe (1) comprenant un corps de pompe (3) définissant une chambre de pompe (13), une paroi mobile (48) du corps de pompe pouvant être déplacée pour modifier le volume de la chambre de pompe, un moyen de sollicitation (53) sollicitant la paroi mobile dans une position d'extension complète dans laquelle le volume de la chambre est maximal, un boîtier (2), des moyens de connexion (20-23, 25-28, 33-36) reliant avec possibilité de détachement le boîtier au corps de pompe, le boîtier comprenant un transducteur piézo-électrique (44) adapté à faire aller et venir la paroi mobile lorsque le boîtier et le corps de pompe sont reliés, les moyens de connexion opérant entre le corps de pompe et le boîtier durant la connexion du corps de pompe au boîtier pour déplacer le corps de pompe depuis une première position relativement au boîtier dans laquelle la paroi mobile se trouve dans sa position d'extension complète en butée avec le transducteur et une seconde position relativement au boîtier dans laquelle la paroi mobile se trouve dans sa position d'extension partielle correspondant à un fonctionnement normal de sorte que la paroi mobile soit sollicitée en contact avec le transducteur par l'action du moyen de sollicitation de manière que le contact soit maintenu durant le va-et-vient du transducteur, caractérisée en ce que les moyens de connexion comprennent des moyens de came (25-28, 33-36) et au moins un élément de verrouillage (20-23) relié au boîtier et mobile entre une position non verrouillée et une position de verrouillage dans laquelle une portion de pied (25-28) de l'élément de verrouillage recouvre une surface de contact (29-32) du corps de pompe afin d'empêcher que le corps de pompe se déconnecte du boîtier, et dans laquelle au moins une des portions de pied et la surface de contact comprennent une surface inclinée (29-32) de manière que le déplacement de l'élément de verrouillage dans la position de verrouillage ait un effet de came de sorte que la portion de pied et la surface de contact constituent conjointement le moyen de came.

2. Pompe selon la revendication 1, comprenant une pluralité d'éléments de verrouillage qui sont espacés périphériquement par rapport à la paroi mobile, les éléments de verrouillage étant montés sur un élément de fixation (24) afin de pouvoir être déplacés à l'unisson entre leurs positions respectives de verrouillage et de déverrouillage.

3. Pompe selon la revendication 2, dans laquelle l'élément de fixation comprend un rebord annulaire (39) et le boîtier comprenant en outre un moyen de guidage annulaire (37) coopérant avec le rebord pour faciliter la rotation axiale du rebord annulaire entre les positions correspondant aux positions de verrouillage et de déverrouillage des éléments de verrouillage.

4. Pompe selon l'une quelconque des revendications précédentes, dans laquelle le corps de pompe est doté d'au moins une ouverture (15-18) dans laquelle un élément de verrouillage coopérant s'étend lorsque le boîtier est relié au corps de pompe.

5. Pompe selon l'une quelconque des revendications précédentes, dans laquelle la pompe mobile est faite d'un matériau élastomère et comprend une portion centrale relativement épaisse (52) qui est relativement rigide et une portion de membrane annulaire relativement flexible (53) reliée périphériquement à la portion ririgide, la portion de membrane ayant une mémoire de forme de manière que la portion centrale soit sollicitée dans la position d'extension complète afin de constituer le moyen de sollicitation.
